# EUROPEAN PATENT SPECIFICATION

(11) **EP 0 760 360 B1**
(45) Date of publication and mention of the grant of the patent: **04.04.2001**
(21) Application number: 96306257.5
(22) Date of filing: 29.08.1996
(51) Int. Cl.: C07C 209/26, C07C 211/48

(54) **Method of manufacturing alkylaniline compounds**
Verfahren zur Herstellung von Alkylanilin-Verbindungen
Procédé pour la préparation de composés d'alkylaniline

(30) Priority: 01.09.1995 JP 22520595
(43) Date of publication of application: 05.03.1997
(73) Proprietor: KONICA CORPORATION, Tokyo 163 (JP)
(72) Inventor: Kaneko, Manabu, c/o Konica Corp., Hino-shi, Tokyo (JP); Tanaka, Shinri, c/o Konica Corp., Hino-shi, Tokyo (JP)
(74) Representative: Ellis-Jones, Patrick George Armine

(56) References cited:
- EP-A- 0 212 303
- EP-A- 0 491 968
- DE-B- 1 014 547
- US-A- 3 261 869
- DATABASE WPI Week 8131 Derwent Publications Ltd., London, GB; AN 81-56703d XP002019724 & SU 781 200 A (AS USSR CHEM PHYS) , 26 November 1980
- DATABASE WPI Week 7335 Derwent Publications Ltd., London, GB; AN 73-49352u XP002030807 & JP 47 038 945 A (SUMITOMO CHEMICAL CO LTD)

## Description

The present invention relates to a method of manufacturing aniline compounds using nitrobenzene compounds and aldehyde compounds.

### BACKGROUND OF THE INVENTION

Alkylaniline compounds, which are prepared using the method of the present invention, are used as an intermediate useful in various fields of industry, For example, they are used as antioxidants, color developing agents, etc.

A conventional method of synthesizing alkyl aniline compounds is a method of reacting a corresponding aniline compound, an organic halide compound and an alkali in an organic solvent. Other methods include a method of reacting the aniline compound and an organic halide compound in the presence of an aqueous phosphoric acid solution and an iodide(cf. Japanese patent O.P.I. Publication No.3-181447(1991)); a method of reacting p-toluene sulfonic ester and a corresponding aniline compound(Japanese Patent O.P.I. Publication No.51-95849(1976); and a method of reacting an aniline compound with a sulfonic ester(Japanese Patent O.P.I. Publication No.3-200758(1991). A further method of synthesizing an objective compound by a catalytic reduction method under the normal pressure is known from pages 174 - 255, vol.4 of "Organic Reactions" published from John Wiley and Sons. Inc.

However, in the method of reacting an aniline compound and an organic halide compound, there has been a problem that the organic halide compound is rather expensive as a raw material, and that too long a time is required for the reaction.

Further in the method of reacting an aniline compound with a p-toluene sulfonic ester or a sulfonic ester derivative, again, there has been a problem that the sulfonic ester compounds are rather expensive as raw materials, and that the pollution load of the released sulfonic acid is large. Still further, most of the aniline compounds are produced by reduction of corresponding nitrobenzene compounds, and the alkylaniline compounds are synthesized from nitrobenzene compounds through two synthesizing steps.

In contrast, in the method of reacting nitrobenzene compounds and aldehyde compounds by a catalytic reduction method under normal pressure, the aldehyde compound used as the raw material is inexpensive in comparison with the corresponding organic halide compound, and, in addition, there is an advantage that this method requires only one process. However, since a long period of time is necessary for the reaction, and the yield of the reaction is rather low, this method has not been considered to be preferable as a method of manufacturing alkylaniline compounds at low cost.

EP-A-0 212 303 relates to a process for the preparation of aromatic dialkyl amines by reacting an aldehyde with a nitrobenzene or an aniline starting material. In the process of EP-A-0 212 303, the aldehyde is used in an amount of from 2 to 10, preferably from 2.5 to 3.5 mols. When the other starting material in this reaction is nitrobenzene, stoichiometry dictates that the amount of aldehyde is from 4 to 20 cools, preferably from 5 to 7 mols per mol of nitrobenzene.

Accordingly, an object of the present invention is to provide a method of producing N,N-dialkylaniline or N-alkylaniline compounds from nitrobenzene compounds and aldehyde compounds through a single step with high yield.

### SUMMARY OF THE INVENTION

The above-mentioned object of the present invention is achieved by the following items:
(1) A method of manufacturing N,N-dialkylaniline represented by Formula III which method comprises:
   (a) mixing a nitrobenzene compound represented by Formula I, an aldehyde compound represented by Formula II and a metal catalyst, wherein the amount of aldehyde compound is from 1 to 3 mol per mol of the nitrobenzene compound, and
   (b) reacting the mixture while applying hydrogen pressure and maintaining a reaction temperature of from 40 to 160 °C: 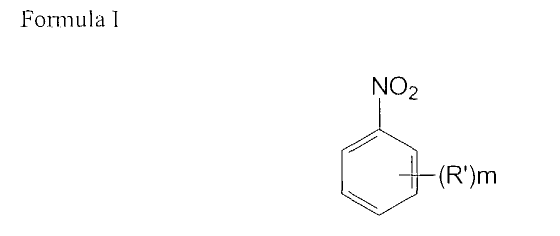
      wherein R¹ represents an alkyl group, an aryl group, an alkoxy group, an aryloxy group, an acylamino group, a carboxyl group or an alkoxycarbonyl group, m is from 0 to 5, provided that when m is two or more, R' may be either the same or different,

         Formula II R²-CHO
      wherein R² represents a hydrogen atom, an alkyl group or an aryl group, 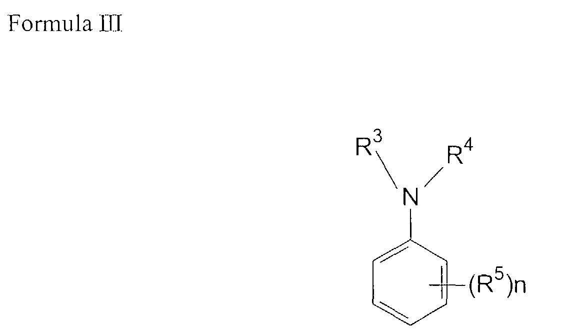
      wherein R³ and R⁴ each independently represent an alkyl group or an aryl group, R⁵ represents an alkyl group, an aryl group, an alkoxy group, an aryloxy group, an acylamino group, a carboxyl group or an alkoxycarbonyl group, n is from 0 to 5, provided that when n is two or more, R⁵ may be either the same or different.
(2) The method of (1) above, wherein the metal catalyst is palladium-carbon.
(3) The method of (1) or (2), wherein the hydrogen pressure is from 0.98 to 14.7 MPa (10 to 150 kg/cm²).
(4) The method of (3), wherein the hydrogen pressure is from 1.96 to 14.7 MPa (20 to 150 kg/cm²).
(5) The method of (4), wherein the hydrogen pressure is from 2.94 to 14.7 MPa (30 to 150 kg/cm²).
(6) The method of (1) to (5), wherein the reaction temperature is from 40°C to 120°C.

For the alkyl group represented by R², a straight or branched alkyl group with from 1 to 13 carbon atoms, including, for example, methyl group, ethyl group, n-propyl group, iso-propyl group, n-butyl group or t-butyl group, is preferable.

Generally, in the case where R² of the aldehyde compound represented by Formula II represents an alkyl group or an aryl group, it is well-known that the aldehyde compound shows a low-reaction activity.

However, when the aldehyde having a low reaction activity is employed in the manufacturing method of the present invention, high production yield can be obtained.

As for the aryl group represented by R², for example, a phenyl group or a naphthyl group can be mentioned.

The alkyl group or the aryl group represented by R² may further have a substituent.

In the present invention, the amount of the aldehyde compound used is from 1 to 3 mol per 1 mol of nitrobenzene compound.

As for the metal catalyst used in the method of the present invention, for example, platinum, palladium or nickel can be mentioned. Preferably, palladium-carbon is used.

The amount of the metal catalyst used when the metal catalyst is a noble metal such as platinum or palladium, is within the range of from 0.01 to 1% and, more preferably, from 0.05 to 0.5% by weight of the nitrobenzene compound. When the metal is other than a noble metal, such as nickel or cobalt, it is used in an amount of from 1 to 10% and more preferably from 2 to 7% by weight of the nitrobenzene compound.

A solvent is optionally used in the reaction. There is no specific limitation as to the solvent which may be used. Examples of suitable solvents include non-polar protic solvents including alcohols, for example, methanol, ethanol, isopropylalcohol; ethers; aromatic hydrocarbons; aliphatic hydrocarbons; esters and dimethylformamide; water or a mixture of these solvents. A preferable amount of the solvent to be used in the reaction is from 0.5 to 20 times and more preferably from 2 to 10 times by weight of the nitrobenzene compound.

The term "hydrogen pressure" as used herein denotes that reaction pressure by hydrogen is usually from 0.92 to 14.7 MPa (10 to 150 kg/cm²), preferably from 1,96 to 14.7 MPa (20 to 150 kg/cm²) and more preferably from 2.94 to 14.7 MPa (30 to 150 kg/cm²).

The process of the present invention is carried out at a temperature of from 40 to 160°C, and preferably from 40 to 120°C.

Time required for the reaction varies depending on the nature of the compound to be used, however, in the present invention the reaction may be carried out for a period generally of from 30 minutes to 10 hours and, preferably within a period of from 1 to 8 hours.

Using the manufacturing method of the present invention, N,N-dialkylaniline represented by Formula III or N-alkylaniline compounds represented by Formula IV can be manufactured. 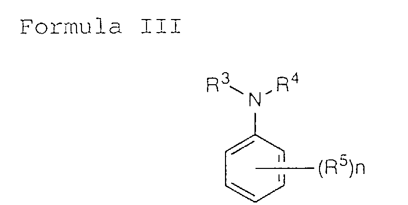 wherein R³ and R⁴ each independently represent an alkyl group or an aryl group, R⁵ represents an alkyl group, an aryl group, an alkoxy group, an aryloxy group, an acylamino group, a carboxyl group or an alkoxycarbonyl group, n is from 0 to 5, provided that when n is two or more, R⁵ may be either the same or different.

Below, representative examples of the alkylaniline compounds manufactured by the manufacturing method of the present invention are shown, however, the scope of the present invention is not limited by these compounds.

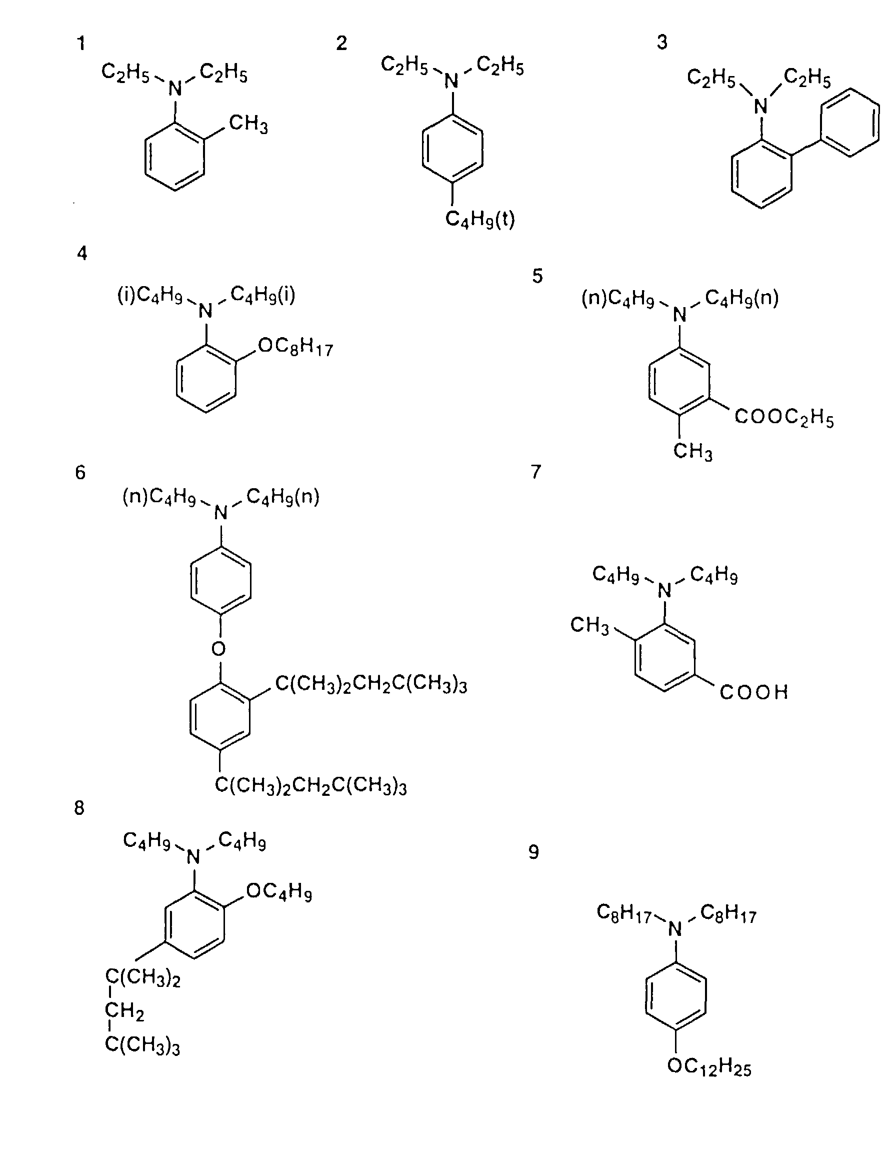

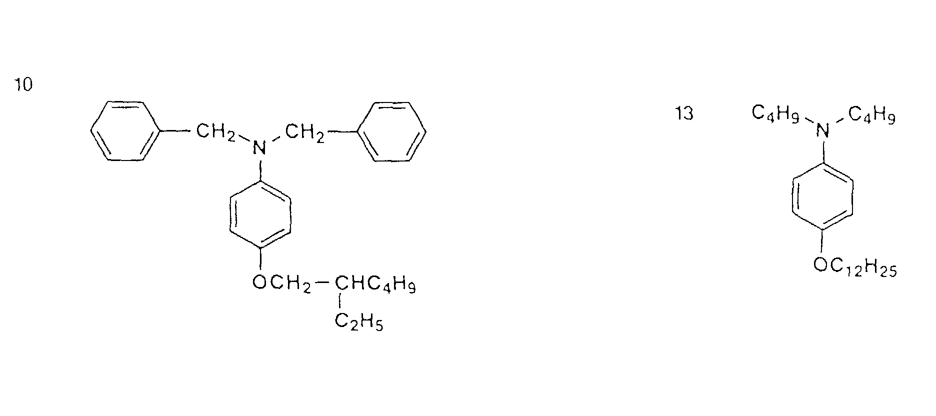

### EXAMPLE

Below, the specified examples of the present invention are explained, however, the scope of the present invention is not limited by these.

Nitrobenzene derivative (a)

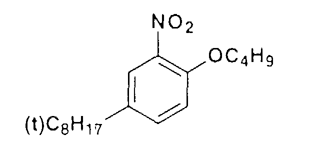

### Example 1

### Synthesis of Exemplified Compound 8

107.6 g (0.35 mol) of the above-mentioned nitrobenzene derivative (a), 75.7 g (1.05 mol) of n-butyl aldehyde, 6.7 g of 5% palladium-carbon (water content 52%), and 430 cc of ethanol were put into an electromagnetic stirring 1 liter autoclave, and after gas substitution with nitrogen, pressurized at 2.94 MPa (30 kg/cm²) with hydrogen gas. This was heated to 80°C under stirring and was subjected to reaction for 5 hours, while maintaining the hydrogen pressure at 2.94 MPa (30 kg/cm²). After reaction was completed, palladium-carbon was removed and the solvent used in the reaction was concentrated by evacuation. Production yield of the object Compound 8 in the reaction solution by using liquid chromatography method was 95.9%. Then this was subjected to vacuum distillation, to obtain 124.5 g of Exemplified Compound 8 (yield: 91.3%). The obtained product was a yellowish oil with boiling point of from 148 to 152°C (40 Pa, 0.3 mmHg). Purity obtained by means of liquid chromatography turned out to be 98.1%. The structure of the object Compound 8 was determined by NMR spectrum and mass spectrum.

### Comparative Example 1

Reaction was carried out under the same conditions as in Example 1, except that in this comparative example, the reaction was carried out at a temperature of from 20 to 40°C, under normal pressure without pressurization with hydrogen. However, Exemplified Compound 8 was not obtained at all.

### Example 1'

Reaction was carried out in the same manner as in Example 1, except that in this example, hydrogen pressure was made to be 590 kPa (6 kg/cm²).

The production yield of Exemplified Compound 8 measured by liquid chromatography, was 62.0%.

### Inventive Example 2

Example 2 was prepared in the same manner as in Example 1, except that hydrogen pressure is changed to 1.96 MPa (20 kg/cm²).

### Comparative sample 2

Comparative sample 2 was prepared in the same manner as in Example 1, except that hydrogen pressure is 421 kPa (4.3 kg/cm²) and the reaction temperature is 20°C.

### Comparative sample 3

Comparative sample 3 was prepared in the same manner as in Example 1, except that the hydrogen pressure is 2.94 MPa (30 kg/cm²) and the reaction temperature is 20°C.

### Inventive Example 3, 4 and 5

Inventive Examples 3, 4 and 5 were prepared in the same manner as in Example 1, except that in these Examples, raw materials and the reaction conditions were changed as set out in Table 1.

### Inventive Example 10

Inventive Example 10 was prepared in the same manner as in Example 1, except that the hydrogen pressure is 3.92 MPa (40 kg/cm²) and the reaction temperature is 80°C.

### Inventive Example 11

Inventive Example 11 was prepared in the same manner as in Example 1, except that the hydrogen pressure is 11.8 MPa (120 kg/cm²) and the reaction temperature is 80°C.

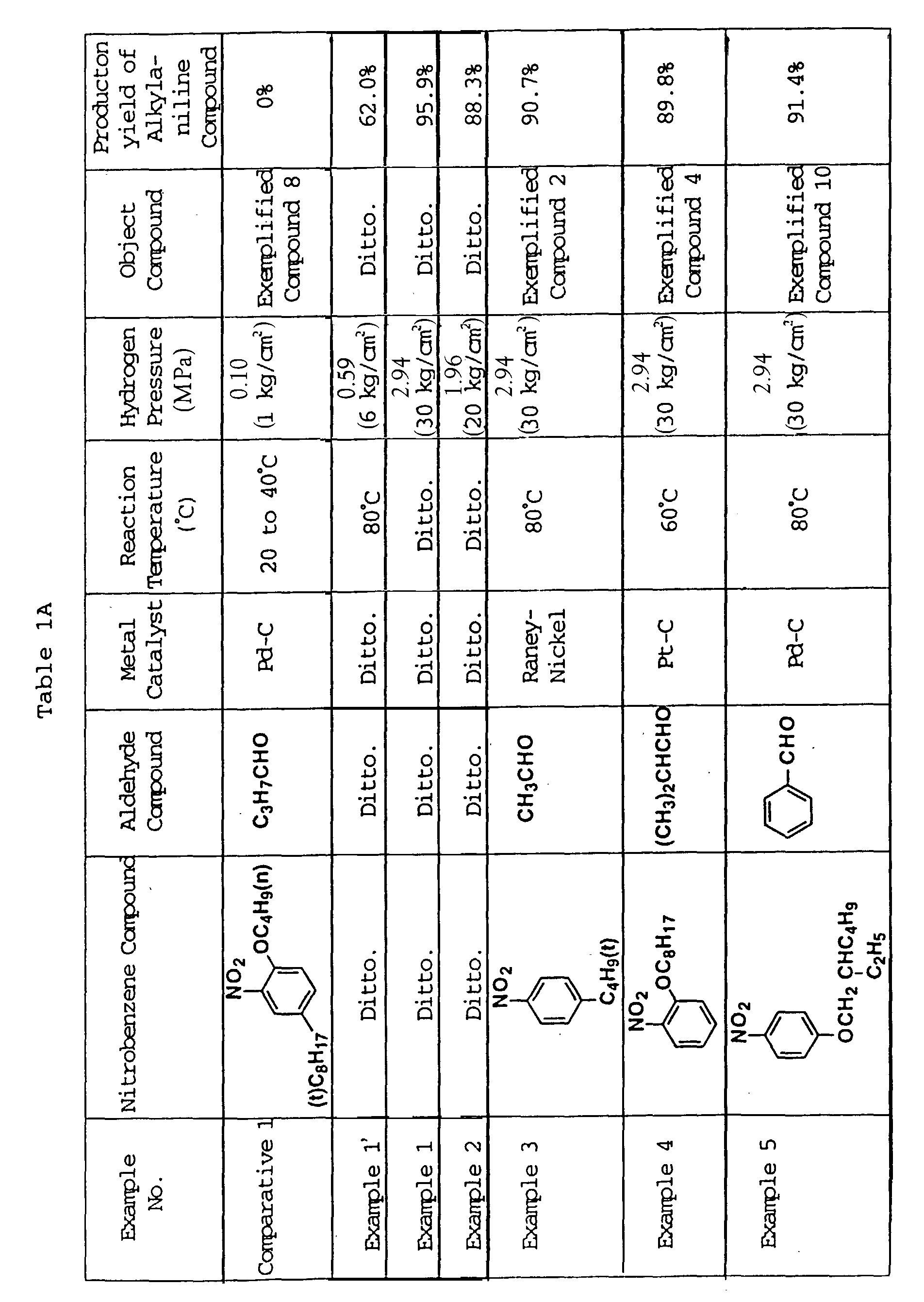

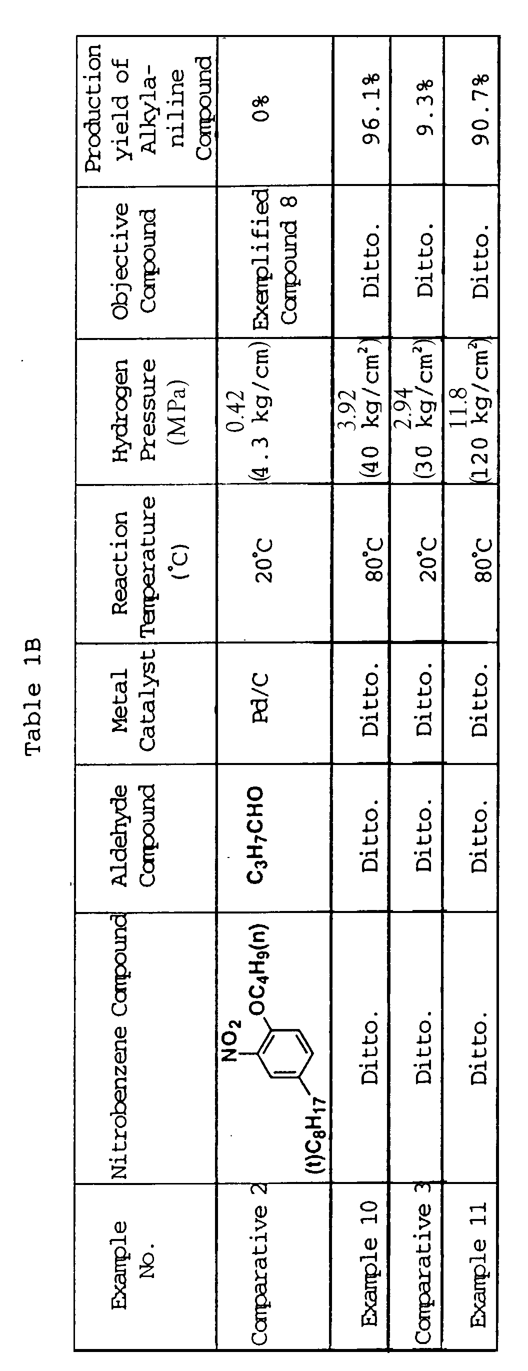

As is apparent from the above results, when using the manufacturing method of the present invention, the object compounds can be synthesized over a short range of time with high yield.

### Example 9

Reaction was carried out in the same manner as Example 1, except that hydrogen pressure was made to be 19.6 MPa (200 kg/cm²). Then, since the reaction was rapidly accelerated and the reaction temperature is rapidly raised up to 150°C, it became impossible to control the reaction temperature, immediately stirring was stopped, reaction pressure was reduced and the reaction was stopped. Production yield of Exemplified Compound 8 was 7.5%, and other complicated by-products and nitrobenzene derivative (a) of raw material were obtained.

### Comparative example 10

In Example 1, the reaction was carried out in a similar manner to Example 1, except that the reaction temperature was made to be 200°C. Production yield of Exemplified Compound 8 was 13.7%, and only other complicated by-products were obtained.

Alkyl radicals R¹, R², R³, R⁴, R⁵, R⁶ and R⁷ include straight chain or branched C₁₋₁₃ alkyl, preferably C₁₋₈, more preferably C₂₋₆ alkyl.

Aryl radicals R¹, R², R³, R⁴, R⁵, R⁶ and R⁷ include C₃₋₁₀ aryl, preferably phenyl or naphthyl.

Also included are alkylaryl radicals such as benzyl.

## Claims

1. A method of manufacturing N,N-dialkylaniline represented by Formula III which method comprises:
(a) mixing a nitrobenzene compound represented by Formula I, an aldehyde compound represented by Formula II and a metal catalyst, wherein the amount of aldehyde compound is from 1 to 3 mol per mol of the nitrobenzene compound, and
(b) reacting the mixture while applying hydrogen pressure and maintaining a reaction temperature of from 40 to 160 °C: 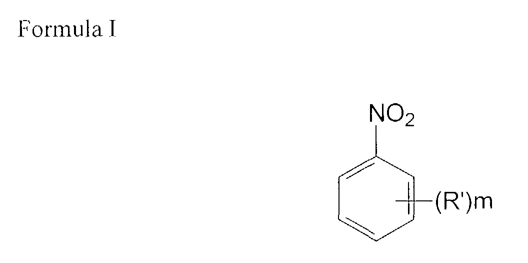
wherein R¹ represents an alkyl group, an aryl group, an alkoxy group, an aryloxy group, an acylamino group, a calboxyl group or an alkoxycarbonyl group, m is from 0 to 5, provided that when m is two or more, R¹ may be either the same or different,
Formula II R²-CHO
wherein R² represents a hydrogen atom, an alkyl group or an aryl group, 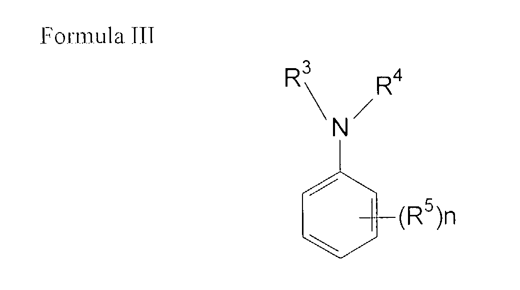
wherein R³ and R⁴ each independently represent an alkyl group or an aryl group, R⁵ represents an alkyl group, an aryl group, an alkoxy group, an aryloxy group, an acylamino group, a carboxyl group or an alkoxycarbonyl group, n is from 0 to 5, provided that when n is two or more, R⁵ may be either the same or different.

2. The method of claim 1, wherein the reaction is carried out in the absence of solvent, or in a solvent chosen from alcohols, ethers and esters.

3. The method of claim 2, wherein the solvent is an alcohol.

4. The method of any preceding claim, wherein R² of Formula II represents an alkyl group or an aryl group.

5. The method of claim 4, wherein R² of Formula II represents a methyl group, an ethyl group, a propyl group, an iso-propyl group, a n-butyl group, or a tert-butyl group.

6. The method of any preceding claim, wherein the reaction temperature is from 40 °C to 120 °C.

7. The method of any preceding claim, wherein the hydrogen pressure is from 0.98 to 14.7 mPa (10 to 150 kg/cm²).

8. The method of claim 7, wherein the hydrogen pressure is from 1.96 to 14.7 mPa (20 to 150 kg/cm²).

9. The method of claim 8, wherein the hydrogen pressure is from 2.94 to 14.7 mPa (30 to 150 kg/cm²).

10. The method of any preceding claim, wherein the metal catalyst is a palladium carbon catalyst.

## Patentansprüche

1. Verfahren zur Herstellung eines N,N-Dialkylanilins der Formel III 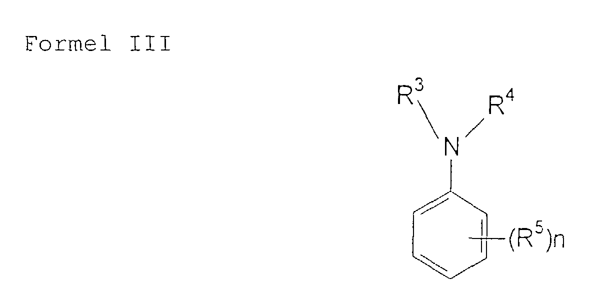 worin bedeuten:
R³ und R⁴ jeweils unabhängig voneinander eine Alkylgruppe oder eine Arylgruppe;
R⁵ eine Alkylgruppe, eine Arylgruppe, eine Alkoxygruppe, eine Aryloxygruppe, eine Acylaminogruppe, eine Carboxylgruppe oder eine Alkoxycarbonylgruppe und
n 0 bis 5, wobei gilt, dass im Falle, dass n = 2 oder mehr, (die verschiedenen Reste) R⁵ gleich oder verschieden sein können,
durch
(a) Vermischen einer Nitrobenzolverbindung der Formel I 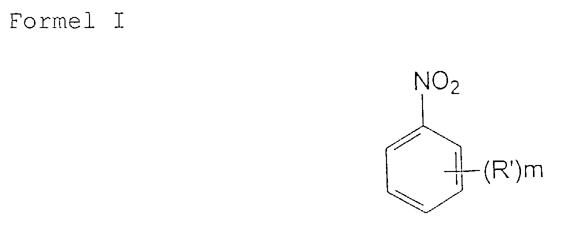 worin bedeuten:
R¹ eine Alkylgruppe, eine Arylgruppe, eine Alkoxygruppe, eine Aryloxygruppe, eine Acylaminogruppe, eine Carboxylgruppe oder eine Alkoxycarbonylgruppe, und
m 0 bis 5, wobei gilt, dass im Falle, dass m = 2 oder mehr, (die verschiedenen Reste) R¹ gleich oder verschieden sein können, mit einer Aldehydverbindung der Formel II
Formel II R²-CHO
worin R² für ein Wasserstoffatom, eine Alkylgruppe oder eine Arylgruppe steht,
und einem Metallkatalysator, wobei die Menge an der Aldehydverbindung 1 bis 3 mol pro mol der Nitrobenzolverbindung beträgt, und
(b) Umsetzen des Gemischs unter Wasserstoffdruck und Aufrechterhalten einer Reaktionstemperatur von 40 - 160 °C.

2. Verfahren nach Anspruch 1, wobei die Reaktion in Abwesenheit eines Lösungsmittels oder in einem aus Alkoholen, Ethern und Estern ausgewählten Lösungsmittel durchgeführt wird.

3. Verfahren nach Anspruch 2, wobei das Lösungsmittel aus einem Alkohol besteht.

4. Verfahren nach einem der vorhergehenden Ansprüche, wobei R² in Formel II für eine Alkylgruppe oder eine Arylgruppe steht.

5. Verfahren nach Anspruch 4, wobei R² in Formel II für eine Methylgruppe, eine Ethylgruppe, eine Propylgruppe, eine Isopropylgruppe, eine n-Butylgruppe oder eine tert.-Butylgruppe steht.

6. Verfahren nach einem der vorhergehenden Ansprüche, wobei die Reaktionstemperatur 40 °C bis 120 °C beträgt.

7. Verfahren nach einem der vorhergehenden Ansprüche, wobei der Wasserstoffdruck 0,98 - 14,7 mPa (10 - 150 kg/cm²) beträgt.

8. Verfahren nach Anspruch 7, wobei der Wasserstoffdruck 1,96 - 14,7 mPa (20 - 150 kg/cm²) beträgt.

9. Verfahren nach Anspruch 8, wobei der Wasserstoffdruck 2,94 - 14,7 mPa (30 - 150 kg/cm²) beträgt.

10. Verfahren nach einem der vorhergehenden Ansprüche, wobei der Metallkatalysator aus einem Palladium-Kohle-Katalysator besteht.

## Revendications

1. Méthode de fabrication de N,N-dialkylaniline représentée par la formule III, cette méthode comprenant le fait :
(a) de mélanger un composé nitrobenzène représenté par la formule I, un composé aldéhyde représenté par la formule II et un catalyseur métallique, la quantité de composé aldéhyde étant de 1 à 3 moles par mole du composé nitrobenzène, et
(b) de faire réagir le mélange tout en appliquant une pression d'hydrogène et en maintenant d'une température de réaction de 40 à 160 °C : 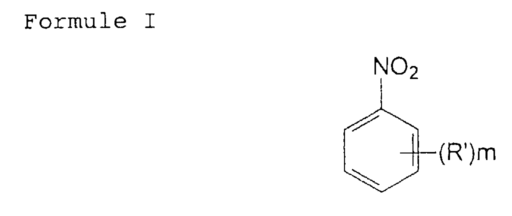 dans laquelle R¹ représente un groupe alkyle, un groupe aryle, un groupe alcoxy, un groupe aryloxy, un groupe acylamino, un groupe carboxyle ou un groupe alcoxycarbonyle, m est de 0 à 5, étant entendu que lorsque m est deux ou plus, les R¹ peuvent être identiques ou différents,
Formule II R²-CHO
dans laquelle R² représente un atome d'hydrogène, un groupe alkyle ou un groupe aryle, 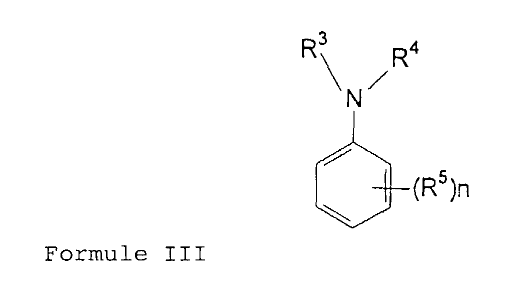 dans laquelle R³ et R⁴ représentent chacun indépendamment un groupe alkyle ou un groupe aryle, R⁵ représente un groupe alkyle, un groupe aryle, un groupe alcoxy, un groupe aryloxy, un groupe acylamino, un groupe carboxyle ou un groupe alcoxycarbonyle, n est de 0 à 5, étant entendu que lorsque n est deux ou plus, les R⁵ peuvent être identiques ou différents.

2. Méthode selon la revendication 1, dans laquelle la réaction est effectuée en l'absence de solvant, ou dans un solvant choisi parmi les alcools, les éthers et les esters.

3. Méthode selon la revendication 2, dans laquelle le solvant est un alcool.

4. Méthode selon l'une quelconque des revendications précédentes, dans laquelle R² de la formule II représente un groupe alkyle ou un groupe aryle.

5. Méthode selon la revendication 4, dans laquelle R² de la formule II représente un groupe méthyle, un groupe éthyle, un groupe propyle, un groupe isopropyle, un groupe n-butyle ou un groupe tert-butyle.

6. Méthode selon l'une quelconque des revendications précédentes, dans laquelle la température de réaction est de 40 °C à 120 °C.

7. Méthode selon l'une quelconque des revendications précédentes, dans laquelle la pression d'hydrogène est de 0,98 à 14,7 mPa (10 à 150 kg/cm²).

8. Méthode selon la revendication 7, dans laquelle la pression d'hydrogène est de 1,96 à 14,7 mPa (20 à 150 kg/cm²).

9. Méthode selon la revendication 8, dans laquelle la pression d'hydrogène est de 2,94 à 14,7 mPa (30 à 150 kg/cm²).

10. Méthode selon l'une quelconque des revendications précédentes, dans laquelle le catalyseur métallique est un catalyseur palladium carbone.
